(19) Europäisches Patentamt
European Patent Office
Office européen des brevets

(11) **EP 2 178 581 B1**

(12) **EUROPÄISCHE PATENTSCHRIFT**

(45) Veröffentlichungstag und Bekanntmachung des Hinweises auf die Patenterteilung:
**17.07.2013 Patentblatt 2013/29**

(51) Int Cl.:
*A61M 1/10* (2006.01)   *F01L 9/04* (2006.01)
*H02K 41/02* (2006.01)   *H02K 41/03* (2006.01)
*H02K 41/035* (2006.01)   *F04B 17/00* (2006.01)

(21) Anmeldenummer: 08801602.7

(22) Anmeldetag: **18.08.2008**

(86) Internationale Anmeldenummer:
**PCT/EP2008/006762**

(87) Internationale Veröffentlichungsnummer:
**WO 2009/024308 (26.02.2009 Gazette 2009/09)**

(54) **LINEARANTRIEB UND PUMPSYSTEM, INSBESONDERE KUNSTHERZ**

LINEAR DRIVE AND PUMP SYSTEM, IN PARTICULAR AN ARTIFICIAL HEART

COMMANDE LINÉAIRE ET SYSTÈME DE POMPAGE, NOTAMMENT COEUR ARTIFICIEL

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **17.08.2007 DE 102007039014**

(43) Veröffentlichungstag der Anmeldung:
**28.04.2010 Patentblatt 2010/17**

(73) Patentinhaber:
• **Rheinisch-Westfälische Technische Hochschule Aachen (RWTH)**
**52062 Aachen (DE)**
• **Herz- Und Diabeteszentrum Nrw**
**32545 Bad Oeynhausen (DE)**

(72) Erfinder:
• **FINOCCHIARO, Thomas**
**52074 Aachen (DE)**
• **BUTSCHEN, Thomas**
**41751 Viersen (DE)**
• **LESSMANN, Marc**
**59821 Arnsberg (DE)**
• **HAMEYER, Kay**
**3001 Heverlee (BE)**
• **SCHMITZ-RODE, Thomas**
**52070 Aachen (DE)**
• **STEINSEIFER, Ulrich**
**B-4730 Hauset (BE)**
• **KWANT, Paul Barteld**
**NL-6294 AG Vijlen (NL)**

(74) Vertreter: **Cohausz Hannig Borkowski Wißgott Patent- und Rechtsanwaltskanzlei**
**Schumannstrasse 97-99**
**40237 Düsseldorf (DE)**

(56) Entgegenhaltungen:
**DE-A1- 10 360 713    US-A- 5 300 111**
**US-B1- 6 600 399**

**Beschreibung**

Die Erfindung betrifft einen elektrischen Linearantrieb, insbesondere für ein Pumpsystem von einem Kunstherz, mit einem beweglichen und einem stationären Teil, bei dem der stationäre Teil durch eine Permanent-Magnetanordnung und der bewegliche Teil durch eine Spulenanordnung gebildet ist oder umgekehrt und bei dem die Spulenanordnung und die Permanent-Magnetanordnung relativ zueinander durch Bestromung der Spulenanordnung in einer axialen Richtung hin- und herbewegbar sind, die Spulenanordnung in einem sich in einer axialen Richtung erstreckenden Luftspalt der Permanent-Magnetanordnung angeordnet ist, deren magnetisches Material in axialer Richtung magnetisiert ist und die zumindest an ihren beiden axialen Enden innere und äußere Polschuhe aufweist, die einander in radialer Richtung und durch den Luftspalt beabstandet gegenüberliegen, wodurch das magnetische Feld im Luftspalt im Bereich der jeweils einander gegenüberliegenden Polschuhe in radialer Richtung konzentriert ist, wodurch wenigstens ein magnetischer Kreis ausgebildet ist, bei dem die Magnetfeldlinien sowohl durch den Luftspalt radial von innen nach außen als auch durch den Luftspalt von außen nach innen verlaufen.

Ein solcher Linearantrieb ist z.B. aus der Publikation DE 103 60 713 A1 bekannt.

Die Erfindung betrifft weiterhin ein Pumpsystem mit einem solchen Antrieb, insbesondere ein Kunstherz zur intra- oder extracorporalen Unterstützung bzw. den Ersatz von Organen, insbesondere des Herzen.

Soweit in dieser Erfindungsbeschreibung Merkmale zu einem Antrieb genannt sind, gelten diese Merkmale auch für ein Pumpsystem, insbesondere für ein Kunstherz und umgekehrt.

Beispielsweise kann ein solches Pumpsystem als Kunstherz intracorporal das Herz ersetzen und somit Patienten, die am Herzen erkrankt sind und bislang noch überwiegend auf ein Spenderherz angewiesen sind, das Leben retten. Dabei bleibt im Gegensatz zu anderen, bislang standardmäßig eingesetzten Systemen, die Lebensqualität des Patienten zu einem hohen Anteil erhalten.

Herzunterstützungssysteme sind im Stand der Technik allgemein bekannt. Eine Vielzahl von Herzunterstützungssystemen sind bereits seit den 20er Jahren entwickelt worden und werden mit wachsendem Erfolg beim Menschen eingesetzt. Hierbei wird generell zwischen herzunterstützenden Systemen und herzersetzenden Systemen unterschieden.

Beim Einsatz herzunterstützender Systeme ist das Herz in seiner Funktionalität eingeschränkt, kann jedoch noch einen Teil seiner ursprünglichen Pumpleistung weiterhin erfüllen. Einer oder beiden Herzkammern wird ein künstliches Pumpsystem parallel oder in Serie geschaltet, welches das Herz unterstützt. Damit kann unter Umständen eine Erholung des Herzens erzielt und das System dann wieder entfernt werden.

Ist das natürliche Herz so stark geschwächt, dass eine Herzunterstützung nicht ausreicht, um den Körper hinreichend mit Blut zu versorgen und eine Erholung des Herzens nicht zu erwarten ist, muss das natürliche Herz entfernt und durch eine Alternative, z.B. ein Kunstherz oder ein transplantiertes Spenderherz ersetzt werden.

Da jedoch die Nachfrage nach Spenderherzen immer weiter steigt und gleichzeitig die Spendebereitschaft sinkt, stehen nicht mehr ausreichend Spenderherzen zur Verfügung. Der Einsatz eines künstlichen Herzens kann die hohe Sterberate der Patienten auf der Warteliste verringern.

Das Incor ist ein typischer Vertreter herzunterstützender System, das bereits beim Menschen eingesetzt wird. Als herzersetzendes System kommt z.B. das Cardiowest zum Einsatz. Die einzelnen herzunterstützenden bzw. herzersetzenden Systeme unterscheiden sich stark in Aufbau und Funktionsweise. Die Systeme lassen sich in kontinuierlich arbeitende Radial- oder Axialpumpen (z.B. Incor) und pulsatile Verdrängungspumpen (z.B. Cardiowest), unterteilen.

Die Kraftübertragung zwischen Antrieb und Pumpmembran wird durch starre mechanische Verbindungen (z.B. Abiocor 2), hydraulisch (z.B. Abiocor 1), pneumatisch (z.B. Cardiowest) oder magnetisch (z.B. Magscrew) umgesetzt. Dabei erfolgt die Kraftübertragung in Form einer Schubkraft oder eines Drehmoments entweder direkt (z.B. Cardiowest) oder mittels einer Untersetzung (z.B. Abiocor 2).

Auf die Verlustwärme des Antriebs, die eine Schädigung des Blutes in Form einer Gerinnung hervorrufen kann, wird spezielles Augenmerk gelegt. Durch teilweise oder vollständige Füllung des Antriebs mit Kühlflüssigkeit, die bei einigen Konzepten gleichzeitig als Schmiermittel für die Lagerung dient, wie z.B. gemäß US 5,300,111, wird versucht, die Verlustleistung möglichst homogen an das Blut und das umliegende Gewebe abzugeben.

Die Lagerung der Pumpsysteme wird durch konventionelle Kugel und Gleitlager (z.B. Abiocor 2) verschleißbehaftet oder durch hydrodynamische Lager, z.B. gemäß US 5,360,445 und Magnetlager weitestgehend verschleißfrei umgesetzt. Der Trend geht hin zu verschleißarmen Antrieben, die mindestens eine Standzeit von 5 Jahren erreichen. Für eine mechanische Entlastung der Lager werden rotationssymmetrische Antriebe verwendet, bei denen sich die Anziehungskräfte zwischen Stator und Rotor bei rotierenden Maschinen bzw. Primärteil und Sekundärteil bei Linearantrieben bei einer Zentrierung kompensieren. Damit fallen die Lager leichter und kompakter aus und erreichen höhere Standzeiten. Da eine ideale Zentrierung technisch nicht umsetzbar ist, entstehen bei allen bisherigen Systemen Anziehungskräfte zwischen Stator und Rotor bzw. Primärteil und Sekundärteil, die zu einem vorzeitigen Verschleiß der Lager führen.

Die meisten Gemeinsamkeiten zwischen der Erfindung und dem Stand der Technik haben die elektrischen Linearantriebe ohne Untersetzung z.B. gemäß US 5,360,445 und US 5,300,111.

Alle bisherigen, oben aufgeführten Konzepte haben nur bei idealer Zentrierung des beweglichen Teils bzgl. dem feststehenden Teil keine resultierende Kraft in radialer Richtung. Da in dem einen Teil stets ferromagnetisches Material, in dem anderen Teil magnetisches Material eingesetzt wird, entstehen zwischen den zwei Teilen stets Anziehungskräfte, die sich nur bei einer idealen Zentrierung kompensieren würden. Da eine ideale Zentrierung technisch nicht realisierbar ist, liegen immer mehr oder weniger stark ausgeprägte Kräfte zwischen den zwei Teilen in radialer Richtung vor. Diese belasten neben der eigentlichen Führungsfunktion die Lager und führen zu einem verstärkten Lagerverschleiß, wodurch die Standzeit reduziert wird.

Bisherige Konzepte maximieren nicht die Kraftdichte. Wie im Patent von Panton (US 5,300,111) beschrieben, ist bei der Entwicklung von Kunstherzen der Abtransport der Wärme an das umliegende Gewebe und das Blut durch geeignete Kühlmaßnahmen realisierbar. Vielmehr sind das Gewicht und die Abmessungen des künstlichen Herzens aufgrund des stark begrenzten Raumes im Brustkorb bei einer vorgegebenen Schubkraft ein Hauptproblem. Zwar wird nach dem im Patent von Goldowsky (US 5,924,975) beschriebenen Bestromungskonzept durch die Wahl gleichlanger Spulen- und Magnetsegmente bei minimalen ohmschen Verlusten die Kraft maximiert, doch schwankt die Kraft wie zuvor beschrieben stark in Abhängigkeit von dem Überdeckungsverhältnis und nimmt nicht den absolut maximalen Wert an.

Ursache hierfür ist eine Aufweitung des magnetischen Feldes (Streuung) in den Bereichen der benachbarten, nicht bestromten Spulen. Somit muss für die Sicherstellung einer vorgegebenen Kraft das Antriebssystem größer ausgelegt werden als ein System, das den Streufluss mit ausnutzen würde.

Das Konzept nach Yeakley (US 6,194,796 B1) nutzt diesen Streufluss durch den Einsatz von nur einem Spulensegment zwar aus, jedoch führt dieses lange Spulensegment zu hohen ohmschen Verlusten, die den Wirkungsgrad stark reduzieren.

Die magnetische Flussdichte im Bereich der Spulensegmente ist bei allen Konzepten maximal so groß wie die von den Magneten erzeugte Flussdichte. Nach Lorentz ist die Kraft auf die stromdurchflossenen Spulensegmente proportional zur magnetischen Flussdichte. Durch eine Vergrößerung der magnetischen Flussdichte, z.B. in Form eines Flusskonzentrators, könnte somit die Kraft bei konstanter ohmscher Verlustleistung deutlich gesteigert werden. Damit wäre eine Erhöhung des Wirkungsgrades oder eine Verkleinerung des Antriebs möglich.

Rotatorische Antriebkonzepte erzeugen nur eine kontinuierliche Strömungsgeschwindigkeit des Blutes, das Pumpverhalten des menschlichen Herzens wird nicht nachempfunden. Ebenso sind bei diesen Konzepten die hohen Rotationsgeschwindigkeiten der Rotoren von Nachteil, durch die hohe Scherkräfte auf das Blut ausgeübt werden, die zu einer verstärkten Schädigung des Blutes führen.

Konzepte nach Vitale (US 6,190,409 B1) sind in der Lage, den Pumpvorgang des menschlichen Herzens in der Pumpleistung nachzubilden, jedoch kann durch den Rotationsmotor innerhalb eines Pumpzyklus der Druck- und Volumenstromverlauf des Blutes nicht ausreichend dem menschlichen Herzen nachgebildet werden.

Des Weiteren arbeiten diese Systeme teilweise mit einer erhöhten Schlagfrequenz.

Beim Abiocor1 kommt Silikonöl zum Einsatz, das den gesamten Antrieb ausfüllt. Da kein kompressibles Medium (z.B. Luft) im System vorhanden ist, wird die Membran in der Diastole (Füllungsphase) aktiv angesaugt, wodurch es zum hämodynamisch ungünstigen Kollabieren des Vorhofs kommen kann.

Zur Gewährleistung einer gewissen Lebensqualität und Perspektive für den Patienten müssen organunterstützende bzw. organersetzende Systeme möglichst leise sein und eine garantierte Standzeit von 5 Jahren aufweisen. Alle bisherigen Konzepte setzen kraftwandelnde Anordnungen wie mechanische, magnetische oder pneumatische Getriebe ein. Die vielen beweglichen Komponenten erzeugen jedoch eine verstärkte Geräuschentwicklung und einen erhöhten Verschleiß.

Aufgabe der Erfindung ist es, einen Antrieb allgemeiner Art, insbesondere für ein Pumpsystem, insbesondere ein Kunstherz oder Unterstützungssystem bereit zu stellen, der nahezu verschleißfrei arbeitet und so eine möglichst lange Haltbarkeit sicherstellt, so kompakt gebaut ist, dass er beispielsweise auch als Antrieb bei einem Kunstherz auch in kleineren Patienten eingesetzt werden kann und eine auf physiologisches Verhalten einstellbare Pumpfunktion ermöglicht und den Körper möglichst physiologisch, dem natürlichen Herzen entsprechend pulsierend, mit Blut versorgt.

Dabei sollen die zuvor beschriebenen Nachteile der bisherigen Konzepte möglichst umfassend vermieden werden, während ihre Vorteile in einem einzigen System vereint werden.

Besonderheiten an der Erfindung sind u.a. die Verringerung der Baugröße, so dass eine signifikante Steigerung des Patientenkreises ermöglicht wird, dem das System implantiert werden kann. Zudem ermöglicht die Erfindung eine längere System - Lebensdauer als die meisten bisherigen Systeme.

Weiterhin kann ein erfindungsgemäßer Antrieb nicht nur zum Antrieb eines Pumpsystems und hier z.B. als Kunstherz eingesetzt werden, sondern für jegliche Art von Antriebsanwendungen und insbesondere jegliche Art von Pumpanwendungen eingesetzt werden.

Insbesondere liegt hierbei der Fokus auf dem, insbesondere pulsierenden Transport von Flüssigkeiten, z.B. die hochempfindlich gegenüber Drücken, Scherspannungen, Temperaturen, Beschleunigungen etc. sind, wie beispielsweise Blut. Ein bevorzugtes Einsatzgebiet erfindungsgemäßer Antriebe liegt somit auf dem Gebiet von Kunstherzen, ist jedoch nicht hierauf beschränkt.

Gemäß einem Aspekt der Erfindung wird die Aufgabe gelöst durch einen gattungsgemäßen Linearantrieb, der dadurch weitergebildet ist, dass die Permanentmagnetanordnung in radialer Richtung auf beiden Seiten des Luftspaltes zwischen den Polschuhen Permanentmagnete aufweist und die Permanent-Magnetanordnung einen ersten inneren Permanentmagneten und einen koaxial dazu angeordneten und zur Bildung des Luftspaltes beabstandeten zweiten äußeren, insbesondere im Querschnitt rahmen- oder ringförmigen Permanentmagneten aufweist.

Eine derartige erfindungsgemäße Anordnung hat den Vorteil, dass gegenüber üblichen Tauchspulenanordnungen die Spulenanordnungen nicht nur in die Permanentmagnetanordnung bis zu einem gewissen Grad eintauchen kann, sondern dass hier der Luftspalt zu beiden axialen Enden der Permanent-Magnetanordnung offen ist und somit eine Spulenanordnung aus beiden axialen Ende heraus- bzw. in die Enden einfahren kann. Die Verfahrweite eines solchen Antriebes hängt somit im Wesentlichen nur von der axialen Länge der Spulenanordnung ab.

Darüber hinaus ergibt sich durch die Magnetisierung der Permanent-Magnetanordnung in axialer Richtung wenigstens ein magnetischer Kreis, der in axialer Richtung über die Permanentmagnetanordnung verläuft und in radialer Richtung zweimal über die Polschuhe und den Luftspalt, nämlich zumindest an den axialen Enden, wobei an jeder Stelle, an welcher durch die Polschuhe die Magnetfeldlinien in radialer Richtung konzentriert sind, sich ein Luftspalt befindet. Die Spulenanordnung kann also entlang der axialen Länge des Antriebs die wenigstens zwei Bereiche (entlang der axialen Erstrekkung) maximaler Feldlinienkonzentration durchfahren, die sich zumindest an den axialen Enden der Permanentmagnetanordnung befindet. In axialer Richtung zwischen den Polschuhen ist hingegen das Magnetfeld bis auf Streuanteile zumindest im Wesentlichen parallel zur axialen Richtung ausgerichtet.

In einer möglichen einfachen, jedoch nicht erfindungsgemäßen Ausgestaltung des Antriebs kann es vorgesehen sein, dass die Permanent-Magnetanordnung zumindest bereichsweise in axialer Richtung nur einen Permanentmagneten aufweist, der in radialer Richtung auf einer Seite des insbesondere im Querschnitt rahmen- oder ringförmigen Luftspaltes angeordnet ist, wobei auf der anderen Seite des Luftspaltes die Polschuhe durch ein magnetisierbares, nicht selbst magnetisches Material zur Führung der Feldlinien verbunden sind. So kann also z.B. ein Permanentmagnet koaxial innen oder außen bezüglich des insbesondere im Querschnitt rahmen- oder ringförmigen Luftspaltes vorgesehen sein, wobei auf der jeweils anderen Seite lediglich insbesondere im Querschnitt rahmen- oder ringförmige Polschuhe bzw. Jochelemente vorgesehen sind, um die Feldlinien zu führen.

Erfindungsgemäß ist es hingegenvorgesehen, dass die Permanentmagnetanordnung in radialer Richtung auf beiden Seiten des insbesondere im Querschnitt rahmen- oder ringförmigen Luftspaltes zwischen den Polschuhen Permanentmagnete aufweist. Hierbei hat die Permanent-Magnetanordnung einen ersten inneren Permanentmagneten (ggfs. mit innerem insbesondere koaxialem Durchgang) und einen koaxial dazu angeordneten und zur Bildung des Luftspaltes beabstandeten zweiten äußeren, insbesondere im Querschnitt rahmenoder ringförmigen Permanentmagneten aufweisen. Dabei weisen die jeweiligen axialen Enden von innerem und äußerem Permanentmagnet unterschiedliche Polarität auf.

Eine erfindungsgemäße Antriebsanordnung weist somit also zumindest zwei Paare von radial einander gegenüberliegenden und über den Luftspalt beabstandete Polschuhe auf, wobei jedes Paar an einem axialen Ende angeordnet ist. Bevorzugt ist dabei die Querschnittsform eines Polschuhs an die Querschnittform des jeweiligen Permanentmagneten angepasst, mit dem der Polschuh verbunden ist.

Es kann in einer anderen Ausführung auch vorgesehen sein, dass zwischen den axial endseitigen Polschuhen wenigstens ein weiteres Paar von durch den Luftspalt beabstandeten und in radialer Richtung einander gegenüberliegenden Polschuhen angeordnet ist, die das Magnetfeld in radialer Richtung konzentrieren. Es kann demnach bei einer solchen Anordnung nicht nur zwei radiale Übergänge der Magnetfeldlinien geben sondern auch drei oder mehr, wodurch in axialer Richtung hintereinander liegend auch zwei oder mehr magnetische Kreise gebildet werden können und in jedem der Kreise die Magnetfeldlinien in radialer Richtung einmal von innen nach außen durch den Luftspalt und einmal von außen nach innen durch den Luftspalt verlaufen. Dabei haben zwei in axialer Richtung benachbarte Magnetkreise jeweils ein gemeinsames Polschuhpaar.

Insbesondere liegen bei einer solchen Permanentmagnetanordnung in axialer Richtung wenigstens zwei Permanentmagnete hintereinander, die durch einen Polschuh getrennt sind, was sowohl für die koaxial innenliegende wie auch die koaxial außenliegende Permanetmagnetanordnung gilt oder nur für eine der Permanentmagnetanordnungen, wenn gemäß der erstgenannten Ausführung auf der gegenüberliegenden Seite Jochmaterial angeordnet ist.

Die Permanentmagnete der Permanent-Magnetanordnung weisen in axialer Richtung wenigstens einen Polarisierungswechsel auf. Grundsätzlich sind beliebig viele Polarisierungswechsel möglich.

Mit dem hier beschriebenen Antrieb kann bevorzugt ein Pumpsystem für ein Kunstherz ausgebildet werden.

Bei allen Ausführungen eines solchen Antriebs, sei es als Pumpsystem für ein Kunstherz oder auch für beliebige anderen Antriebsanwendungen, kann es in einer Weiterbildung auch vorgesehen sein, dass die Dicke der Polschuhe in radialer Richtung auf den Luftspalt zu zunimmt. Hierdurch kann Material und somit Gewicht eingespart werden. Ein Abnehmen der Dicke in Richtung vom Luftspalt weg ist dabei unschädlich, da die Magnetfeldkonzentration in dieser Richtung ebenso abnimmt aufgrund der Umlenkung der Magnetfeldlinien aus der axialen in die radiale Richtung am Ort der Polschuhe.

Da es bei diesen Ausführungen vorgesehen ist, die Spulenanordnung an beiden axialen Seiten der Permanent-Magnetanordnung aus dieser heraus und in diese hinein zu fahren, jedoch entlang der axialen Erstreckung wenigstens zwei Orte gegeben sind, an denen die Magnetfeldlinien in entgegengesetzten radialen Richtungen verlaufen, ist es in einer Weiterbildung vorgesehen, dass die Spulenanordnung an diesen Stellen zur definierten Bewegung in einer gewünschten Richtung während des Betriebs in entgegengesetzten Richtungen stromdurchflossen ist. Hierfür kann es z.B. in einer einfachen Ausgestaltung vorgesehen sein, dass die Spulenanordnung eine einzige Spulenwicklung aufweist, deren Wickelorientierung sich in axialer Richtung der Spulenanordnung wenigstens einmalig umkehrt. So wird bei gleich bleibender Bestromung wegen der Umkehrung der Wicklungsorientierung in der Praxis auch die Umkehrung der Stromrichtung erzielt und somit in axialer Richtung gleichgerichtete Antriebkräfte, die zwischen Permanent-Magnetanordnung und Spulenanordnung wirken.

In einer anderen demgegenüber bevorzugten Ausführung kann es vorgesehen sein, dass die Spulenanordnung wenigstens zwei in axialer Richtung hintereinander liegende und unterschiedlich bestrombare Spulen bzw. Spulensegmente aufweist, wobei, insbesondere durch eine übergeordnete Steuerung nur diejenigen Spulensegmente bestromt sind, die im Bereich der Polschuhe liegen. Eine unterschiedliche Bestromung kann dabei eine unterschiedliche Stromstärke und/oder Stromrichtung bedeuten. Hierbei können die Spulensegmente und/oder Polschuhe in axialer Richtung unterschiedlich breit sein.

Bei sämtlichen Ausführungen der Erfindung nach diesem Aspekt ist es vorgesehen, die Elemente wie Permanentmagnete und Polschuhe, die radial innen und außen relativ zum Luftspalt angeordnet sind, fest mechanisch miteinander zu verbinden, damit deren relative Lage zueinander fixiert ist. Dies kann z.B. durch einen umgebenden festen Rahmenaufbau gegeben sein.

Gemäß einem anderen, jedoch nicht erfindungsgemäßen Aspekt wird die Aufgabe auch gelöst durch einen gattungsgemäßen Linearantrieb, der dadurch weitergebildet ist, dass die Permanent-Magnetanordnung als ein Stapel von rahmen-, insbesondere ringförmigen Magneten ausgebildet ist, die in axialer Richtung abwechselnd radial und axial magnetisiert sind, so dass das magnetische Feld auf der einen Rahmen- bzw. Ringseite, insbesondere der radial außen liegenden Seite, verstärkt und auf der anderen Seite, insbesondere der radial innen liegenden Seite, abgeschwächt ist, wobei die Spulenanordnung koaxial zur Permanent-Magnetanordnung auf der Seite des verstärkten Magnetfeldes angeordnet ist. Auch mit einem solchen Antrieb kann wiederum bevorzugt jedoch nicht einschränkend ein Pumpsystem für ein Kunstherz ausgebildet werden.

Nach diesem Aspekt erfolgt zwar eine konstruktiv andere Lösung der gestellten Aufgabe, es wird jedoch gemäß beiden Aspekten von der erfindungswesentlichen Idee Gebrauch gemacht, durch eine besondere Art der Ausgestaltung der magnetischen Polarisierung (Magnetisierungsrichtung) der Permanentmagnetanordnung und konstruktiven Umgebung eine lokale Konzentrierung der Magnetfeldlinien im unmittelbaren Bereich der wirkenden Bestromung der Spulenanordnung zu erzielen. Somit sind beide Aspekte durch denselben Erfindungsgedanken verbunden. Wesentlich ist auch, dass bei beiden Konzepten keine Relativbewegung zwischen Permanentmagnet und Flussführungskomponenten (z.B. Vacoflux) auftritt.

Hierfür kann es z.B. in einer einfachen Ausgestaltung vorgesehen sein, dass die Spulenanordnung eine einzige Spulenwicklung aufweist, deren Wickelorientierung sich in axialer Richtung der Spulenanordnung wenigstens einmalig umkehrt. So wird bei gleich bleibender Bestromung wegen der Umkehrung der Wicklungsorientierung in der Praxis auch die Umkehrung der Stromrichtung erzielt und somit in axialer Richtung gleichgerichtete Antriebkräfte, die zwischen Permanent-Magnetanordnung und Spulenanordnung wirken.

Bei dieser Ausführung kann es ebenso vorgesehen sein, die Spulenanordnung in einzelne Spulensegmente zu unterteilen und, insbesondere durch eine übergeordnete Steuerung, nur die Spulensegmente zu bestromen, die sich mit dem magnetischen Material überdecken und/oder die vorrangig von einem radialen Magnetfeld durchströmt werden. Dabei kann es gleichzeitig vorgesehen sein, die Spulensegmente stromfrei zu schalten, in denen das magnetische Feld im Wesentlichen parallel zur Achse des Linearantriebs verläuft. Auch kann es vorgesehen sein, gleichzeitig die Spulensegmente stromfrei zu schalten, in denen das magnetische Feld nicht die genügende Stärke aufweist. Dabei kann der Übergang zwischen stromlosem und bestromtem Zustand der Spulensegmente lageabhängig, insbesondere kontinuierlich einstellbar sein und z.B. durch eine übergeordnete Steuerung vorgenommen werden.

Bei den Ausführungen zu beiden Aspekten kann es vorgesehen sein, dass eine Stromversorgung der Spulenanordnung z.B. realisiert ist durch Schleifkontakte und besonders bevorzugt durch wenigstens zwei elektrische Leiter, die spiralförmig ausgebildet sind. In einer definierten Lage können die Leiter jeweils ein Spirale bilden, die in einer Eben liegt, wobei sich durch eine Auslenkung der Spulenanordnung gegenüber der Permanentmagnetanordnung dann das innere bzw. äußere Ende der jeweiligen Spirale aus dieser Ebene herausbewegt. Hierdurch liegt der betreffende spiralförmige Leiter auf einem gedachten Kegelmantel.

Dabei können die wenigstens zwei Leiter in einer Ebene einander spiralförmig umgeben und/oder es kann vorgesehen sein, dass wenigstens zwei Leiter in einer Spirale geschichtet mit einer zwischenliegenden Isolierung angeordnet sind. Insbesondere bei einer Schichtung können mehrere in einer Spirale geschichtete Leiter mit einem Versatz in Spiralrichtung beginnen und/oder enden, um vereinfacht die elektrischen Zuleitung an den versetzten Enden anbringen zu können.

Die Ausbildung der Zuleitungen durch spiralförmige Leiter hat den besonderen Vorteil, dass ein Ende dieser Leiter an einem feststehenden Element des Antriebs befestigt sein kann und das jeweilige andere Ende an einem bewegten Teil, z.B. der Spulenanordnung. Bei den relativen Bewegungen der Teile zueinander bewegen sich die spiralförmigen Leiter aus der Spiralebene heraus ohne eine große mechanische Beanspruchung zu erzeugen. Derartig ausgebildete Leiter haben somit eine hohe Lebensdauer, die gerade in der Anwendung bei Kunstherzen wesentlich ist.

Die Anzahl der spiralförmigen Leiter kann auf zwei begrenzt werden, wenn eine Elektronik (Umrichter) zur Bestromung und Umschaltung von Spulensegmenten an der Spulenanordnung selbst angeordnet ist und somit z.B. mitbewegt wird. Es werden dann nur die zwei Zuleitungen zu dieser Elektronik benötigt.

Die Spulenanordnung selbst kann an einem Kraftübertragungselement befestigt sein, das in einem mittleren Bereich, insbesondere koaxial in der Permanent-Meganetanordnung gelagert ist und sich durch die Permanent-Magnetanordnung hindurch erstreckt. So ist eine Platz sparende Anordnung möglich. Die Spulenwicklungen können z.B. an einem Trägerelement angeordnet sein, das z.B. topfförmig ausgestaltet ist und an dem Kraftübertragungselement angeordnet ist.

Das Kraftübertragungselement kann axial zu beiden Seiten der Permanent-Magnetanordnung Druckplatten aufweisen, mittels denen eine Kraft auf umgebende Elemente, insbesondere eine Membran einer Pumpkammer ausübbar ist.

Der maßgebliche Stand der Technik und Ausführungsbeispiele der Erfindung sind in den nachfolgenden Figuren dargestellt. Es zeigen:

Figur 1: Ein Kunstherz mit einem Linearantrieb gemäß dem Stand der Technik;

Figur 2: die Anordnung der Permanentmagnete und Polschuhelemente nach dem ersten Aspekt der Erfindung ohne Darstellung der Spulenanordnung;

Figur 3: einen Linearantrieb gemäß dem ersten Aspekt z.B. zur Verwendung in einem Kunstherz;

Figur 4: eine Permanentmagnet- und Spulenanordnung nach dem zweiten, jedoch nicht erfindungsgemäßen Aspekt;

Figur 5: die Prinzipdarstellung der Konzentration der Magnetfeldlinien bei einer Ausführung nach dem zweiten Aspekt;

Figur 6: eine Anordnung aus zwei spiralförmig verlaufenden elektrischen Leitern zur Kontaktierung zwischen der sich relativ zueinander bewegenden Spulenanordnung und Permanentmagnetanordnung.

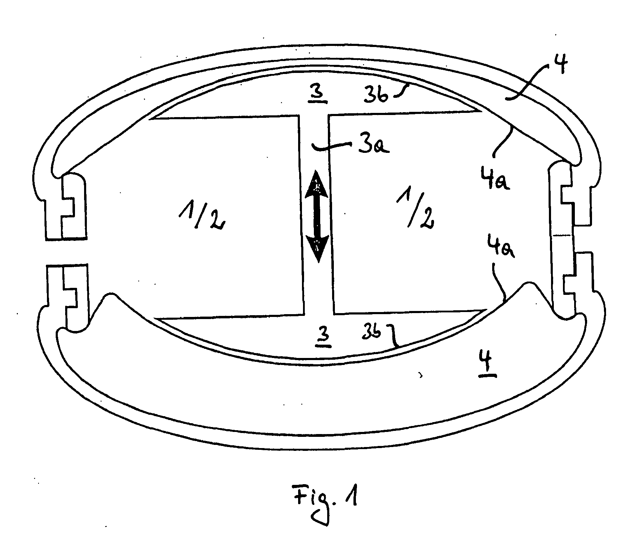

Anhand der Figur 1 werden die Hauptmerkmale einer Pumpanordnung, z.B. für ein Kunstherz, mit einem Linearantrieb erläutert, wobei der Linearantrieb mit seiner Spulen- und Permanentmagnetanordnung hier nicht dargestellt ist. Die hier prinzipiell dargestellte Pumpanordnung kann Linearantriebe gemäß dem ersten erfindungsgemäßen Aspekt und auch gemäß dem zweiten nicht erfindungsgemäßen Aspekt aufnehmen.

Alle linearen Antriebssysteme haben gemeinsam, dass der bewegliche Teil eine Spulenanordnung 1 mit wenigsten einer Spule umfasst und der stationäre Teil wenigstens einen Permanentmagneten 2 oder umgekehrt. Die Permanentmagnete 2 erzeugen ein magnetisches Feld, dessen Flussdichte durch B beschrieben wird. Es durchsetzt die Spulen 1 vollständig oder anteilig. In der Figur 1 sind die Bezugszeichen 1 und 2 lediglich symbolisch an der Stelle angeordnet, die den Antrieb aufnimmt, ohne die Spulen 1 und Magnete 2 konkret zu bezeichnen.

Durch Einprägung eines Stromes I in die Spulen 1 der Spulenanordnung wirkt nach Lorentz auf die Spulen 1 mit der mittleren Windungslänge l und der Windungszahl n eine Kraft F gemäß

$$\vec{F} = n \cdot I \cdot \left(\vec{l} \times \vec{B}\right)$$

Diese Kraft wird in Form eines Kraftübertragungsglieds 3, das mechanisch, hydraulisch, pneumatisch oder magnetisch realisiert wird, auf mindestens eine Pumpkammer 4, bevorzugt auf zwei zu beiden axialen Seiten des Linearantriebs liegende Pumpkammern 4 abwechselnd übertragen. Die Pumpkammern können jeweils eine zum Inneren weisende Membran 4a aufweisen, auf welche das Kraftübertragungsglied 3 bei der Bewegung einwirkt und somit z.B. Blut aus der Kammer 4 auf der einen Seite verdrängt, wobei Blut in die Kammer 4 der anderen Seite einströmt. Damit das Kraftübertragungsglied 3 durch Bestromung der Spulenanordnung bewegt werden kann, ist es vorgesehen, die Spulenanordnung oder die Magnetanordnung an diesem Kraftübertragungsglied 3 zu befestigen, z.B. an einem Wicklungsträger, der am Kraftübertragungslied 3 angeordnet ist.

Die Lagerung der beweglichen Teile erfolgt durch Gleit- oder Kugellager, magnetische Lager oder hydrodynamische Lager, die z.B. am axialen Teil 3a des Kraftübertragungsgliedes 3 wirken. Durch Umkehr der Stromrichtung in den Spulen wird die Richtung der Kraft umgekehrt, so dass eine pulsierende Wirkung auf die Pumpkammern 4 entsteht. Ventile an den Ein- und Auslässen definieren die Strömungsrichtung des Fluids, insbesondere des Blutes. Über die Amplitude des Stroms in den einzelnen Spulen kann die Kraft und damit die Strömungsgeschwindigkeit des Fluids und der Puls geregelt werden.

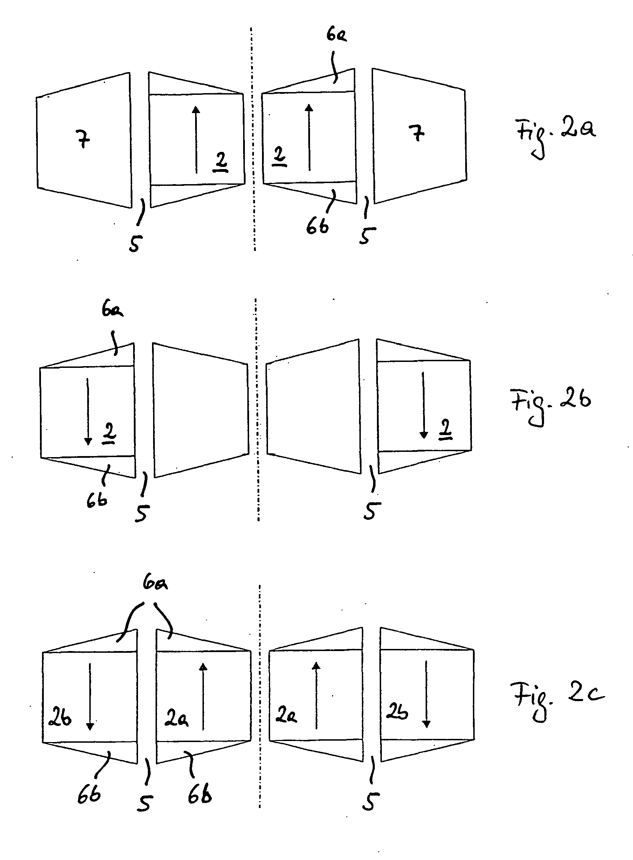

Die Figuren 2 zeigen verschiedene Varianten und eine Ausführungsform gemäß dem ersten Aspekt der Erfindung.

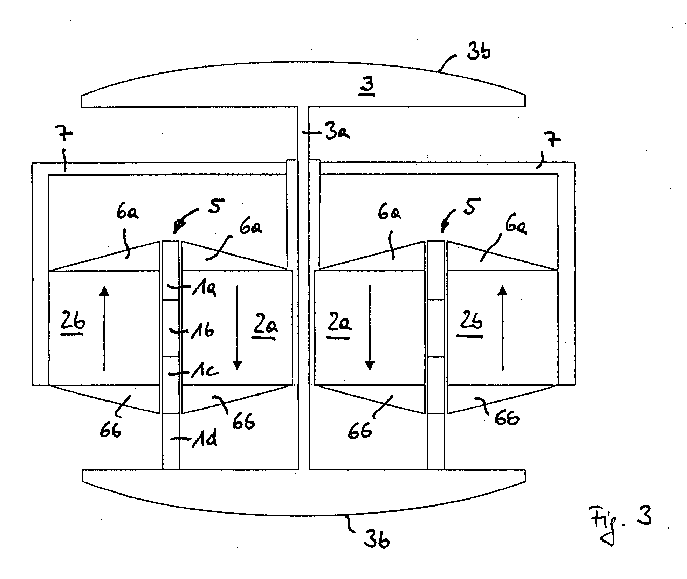

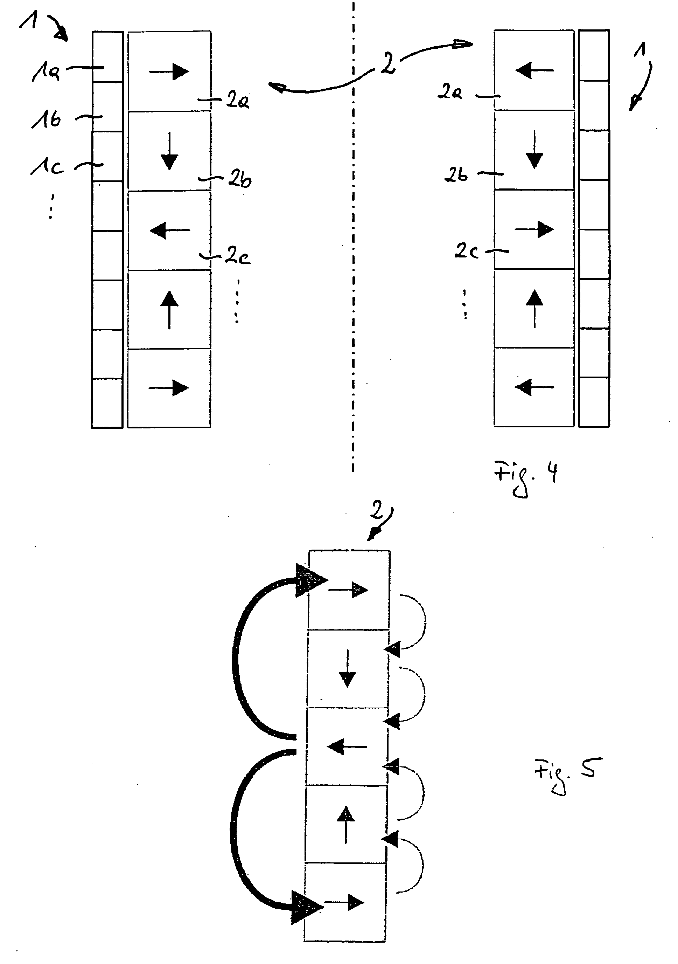

Gemäß Figur 2a ist es in einer nicht erfindungsgemäßen Ausführung vorgesehen, einen bezüglich des Luftspaltes 5 koaxial innen liegenden Permanentmagneten 2 zu verwenden, der eine ausschließlich axiale Magnetisierung aufweist. Das Permanentmagnetmaterial ist in den Figuren 2 durch die Pfeile angedeutet und die Richtung der Magnetisierung durch die Richtung der Pfeile. Hier ist der Permanentmagnet 2 ringförmig mit einem axialen Durchlass, durch den z.B. ein Kraftübertragungsglied 3 hindurchgeführt sein kann und an dem eine entsprechende Lagerung für das Kraftübertragungsglied realisiert sein kann. Beides ist hier zur Vereinfachung in der Darstellung nicht gezeigt. Die gestrichelte Linie zeigt in allen Figuren 2 eine Symmetrieachse, insbesondere eine Rotationssymmetrieachse, die auch bei den Figuren 3 und 4 gegeben ist.

An den beiden axialen (hier oberen und unteren) Enden des Permanentmagneten sind insbesondere im Querschnitt rahmen- oder ringförmigen Polschuhe 6a und 6b vorgesehen und mit dem Permanentmagneten 2 fest verbunden, um das axiale Magnetfeld des Permanentmagneten 2 radial umzulenken und in radialer Richtung zu konzentrieren.

In einem radialen Abstand um den Permanentmagneten 2 herum ist ein rahmen- bzw. ringförmiges Jochelement 7 aus einem magnetisierbaren, jedoch nicht selbst magnetischen Material angeordnet. Durch den Abstand wird der rahmen- oder ringförmige um die Symmetrieachse Luftspalt 5 ausgebildet, der zur Aufnahme einer Spulenanordnung dient, die hier nicht gezeigt ist. Sowohl bei den Polschuhen 6a und 6b wie auch bei dem Jochelement 7 ist die Formgestalt derart, dass deren Dicke in radialer Richtung zum Luftspalt 5 hin zunimmt.

Bezogen auf die Figur 2a ist der Verlauf der Magnetfeldlinien beim inneren Permanentmagneten 2 von unten nach oben, im Bereich des oberen Polschuhs 6a radial von innen nach außen, im Jochelement 7 nach unten und im Bereich des unteren Polschuhs 6b radial von außen nach innen. Es ergibt sich somit ein geschlossener Magnetkreis, bei dem die radial konzentrierten Magnetfeldlinien zweimalig den Luftspalt 5 durchdringen, in jeweils verschiedener Richtung.

Dies ist der wesentliche Unterschied bei dieser Anordnung gegenüber üblichen im Stand der Technik bekannten Tauchspulenanordnungen, bei denen die Spulenanordnung nur in einer Richtung radial von dem Magnetfeld durchsetzt ist.

Die hier nicht gezeigte Spulenanordnung kann somit zu beiden axialen Seiten des Antriebes heraus- und hereingefahren werden.

Gegenüber der Figur 2a ist bei der Figur 2b lediglich die Anordnung von Permanentmagneten 2 mit Polschuhen 6a und 6b und Jochelement 7 vertauscht. Ansonsten ist die Wirkungsweise und die Richtung der Feldlinien identisch.

Eine erfindungsgemäße Ausführung zeigt die Figur 2c, bei der zu beiden Seiten des Luftspaltes 5 Permanentmagnete 2a und 2b angeordnet sind mit rein axialer Magnetisierung, wobei die Magnetisierung von innerem und äußerem Permanetmagneten entgegengesetzt zueinander ist. Beide Permanentmagnete 2a und 2b tragen axial endseitig Polschuhe 6a und 6b, wobei die Polschuhe von innerem und äußerem Magneten 2a / 2b durch den Luftspalt 5 getrennt sind und einander radial gegenüberliegen. Hier ergibt sich dergleiche magnetische Kreis wie bei den Figuren 2a und 2b, jedoch mit größerer Magnetfeldstärke aufgrund der beiden Permanentmagneten. Auch hier ist das Feld axial endseitig in radialer Richtung konzentriert, so dass die hier nicht gezeigte Spulenanordnung 1 im Wesentlichen nur auf der axialen Höhe der Polschuhe vom Magnetfeld durchsetzt ist, in den dazwischen liegenden axialen Bereich jedoch allenfalls nicht zu vermeidende Streufelder wirken, deren Einfluss zumindest im Wesentlichen vernachlässigbar ist.

Die Figur 3 zeigt einen linearen Antrieb mit einer Anordnung der Magnete 2a / 2b und Polschuhepaare 6a / 6b gemäß Figur 2c. Hier befindet sich die Spulenanordnung 1 mit beispielhaft 4 Spulensegmenten 1 a, 1b, 1c und 1d im Luftspalt 5 zwischen den beiden Permanentmagneten 2a und 2b, die jeweils in axialer Richtung und einander entgegengesetzt magnetisiert sind.

Durch den Einsatz von Polschuhen 6, die bevorzugt eine hohe magnetische Sättigungsinduktion aufweisen, wird das Feld im Luftspalt 5 im Bereich der Polschuhe 6 stark konzentriert. Da durch den Einsatz von feldkonzentrierenden Polschuhen 6 im Vergleich zu allen vorherigen Konzepten im Luftspalt ein größeres Feld erzeugt wird, reicht nach

$$\vec{F} = n \cdot I \cdot \left( \vec{l} \times \vec{B} \right)$$

bei konstanter Kraft ein kleinerer Spulenstrom aus. Somit wird die ohmsche Verlustleistung, die proportional zum Quadrat des Stromes ist, stark reduziert: P ist proportional zu $1/B^2$.

Durch den eisenlosen Aufbau des beweglichen bzw. feststehenden Teils entstehen keine maxwellschen Anziehungskräfte zwischen den zwei Teilen. Damit werden die Lager, die die Spulenanordnung 1 über das Kraftübertragungsglied 3 an dem inneren Permanentmagneten 2a lagern, in radialer Richtung entlastet und können kleiner dimensioniert bzw. bezüglich deren Standzeit optimiert werden. Der äußere Magnet 2b ist am inneren Magneten 2a mechanisch befestigt, hier durch die Befestigungselemente 7. Das Kraftübertragungsglied hat hier eine innere Achse 3a, die gelagert ist an der inneren koaxialen Ausnehmung des inneren Magneten 2a. Es ist auch jegliche andere Art der Lagerung möglich. Wesentlich ist lediglich, dass durch eine Bestromung der Spulenanordnung das Kraftübertragungsglied 3 in beiden Richtungen bewegbar ist.

Ein weiterer Vorteil des eisenlosen Aufbaus einer der zwei Komponenten liegt in der Möglichkeit, den einen Teil aus dem anderen Teil herausbewegen zu können, ohne dass maxwellsche Anziehungskräfte zwischen den zwei Komponenten entstehen. Somit kann durch eine Anpassung des Längenverhältnisses zwischen feststehendem und beweglichem Teil das Kraftprofil an ein gefordertes Kraftprofil, z.B. das des menschlichen Herzens, angepasst werden und somit Gewicht und Bauvolumen reduziert werden.

Zur Minimierung der ohmschen Verluste umfasst hier die Spulenanordnung einzeln bestrombare Spulensegmente 1 a, 1 b, 1 c, 1 d (hier als Beispiel 4 Stück), wobei erfindungsgemäß nur diejenigen Segmente bestromt werden, die im Bereich der Polschuhe 6a bzw. 6b liegen. Alle übrigen Spulensegmente tragen nicht zur Kraft bei und können daher stromlos verbleiben.

Für alle möglichen hier gezeigten oder auch nicht gezeigten Ausführungsformen kann die individuelle Bestromung durch eine Steuerung vorgesehen sein, die z.B. direkt an der Spulenanordnung vorgesehen sein kann und von außen lediglich ihre Stromversorgung erhält oder auch extern vom Antrieb angeordnet sein kann. Die Bestromung eines jeweils zwischen den Polschuhen 6 befindlichen Spulensegments kann bei jeder Ausführung z.B. lageabhängig automatisch durch die Steuerung erfolgen. Um die Lage zu ermitteln, kann ein Lagesensor vorgesehen sein, der die Lage der Spulenanordnung und/oder des Kraftübertragungsgliedes relativ zu den Polschuhen bzw. dem Permanentmagneten erfasst. Z.B. kann eine Lichtschranke vorgesehen sein, um eine definierte Nullposition zu erfassen und ein Bewegungssensor zur Erfassung der Bewegungsrichtung und -weite, wie er z.B. auch aus optischen Computermäusen bekannt ist. Ebenso kann z.B. die Positionserfassung direkt duch die Regelung sensorlos erfolgen.

Indem die Spulensegmente und Polschuhe in axialer Richtung unterschiedlich breit gewählt werden, kann ebenfalls der Streufluss zur Erzeugung einer Schubkraft verwendet werden.

Der hier vorgestellte Antrieb kann bei Verwendung in einer Pumpanordnung, wie sie Figur 1 zeigt, den Pumpzyklus des menschlichen Herzens in allen erwähnten Aspekten ideal nachbilden. Es kann dabei eine aktive Ansaugung der Membran 4a während der Diastole vermieden werden, wenn das Kraftübertragungsglied 3 mit seinen Druckplatten 3b die Membran 4a nur lose kontaktiert. Die Lagerung des beweglichen Teils bzgl. des feststehenden Teils kann beispielsweise über konventionelle Lager wie Kugel oder Gleitlager erfolgen, oder vollständig verschleißfrei über hydrodynamische Lager oder Magnetlager.

Die Figur 4 zeigt eine Antriebsanordnung aus Permanentmagnet 2 und Spulenanordnung 1 gemäß dem zweiten, jedoch nicht erfindungsgemäßen Aspekt. Hier ist das magnetische Material der Magnetanordnung 2 als Kombination aufeinanderfolgender zueinander senkrecht stehender Magnetisierungsrichtungen realisiert, insbesondere mehrfach in axialer Richtung abwechselnd axial und radial. Z.B. können Permanentmagnetringe 2a, 2b, 2c, ..... mit abwechselnd zueinander axialer und radialer Magnetisierung aufeinander geschichtet sein. Damit ergibt sich der in Figur 5 dargestellte Feldlinienverlauf, bei dem das Feld auf der linken (äußeren) Seite des magnetischen Materials 2 verstärkt und auf der anderen (inneren) Seite abgeschwächt wird. Durch entsprechend andere Orientierung der Magnetringe kann dies auch umgekehrt erzielt werden.

Es ist dann vorgesehen, die Spulenanordnung 1 im Bereich des verstärkten Magnetfeldes, in dieser Ausführung radial außen, anzuordnen. Es entstehen keine maxwellschen Anziehungskräfte zwischen der Magnetanordnung 2 und den Spulen 1, wodurch eine mechanische Entlastung der Lager in radialer Richtung erreicht wird. Auch hier kann ein Kraftübertragungsglied wie in Figur 1 gezeigt die Spulenanordnung 1 tragen und am inneren freien Durchgang durch die Magnetanordnung 1 gelagert sein.

Durch den weiterhin bevorzugten Einsatz eines dünnen, hier nicht gezeigten Rückschlussjochs innerhalb der Magnetanordnung 2 kann das Streufeld weiter verringert und das magnetische Feld im Bereich der Spulen weiter erhöht werden. Auch dies hat keine Auswirkung auf die Lagerbelastung in radialer Richtung.

Ebenso kann aufgrund der nicht vorhandenen maxwellschen Anziehungskräfte der bewegliche Teil aus dem feststehenden Teil herausgeführt werden, ohne dass rückstellende Kräfte auftreten. Damit ist es möglich, den Antrieb durch Wahl eines geeigneten Längenverhältnisses von magnetischem Material und Spulen an ein gefordertes Kraftprofil, z.B. das eines Kunstherzens, anzupassen.

Durch eine bevorzugte Aufteilung der Spulenanordnung 1 in einzelne Segmente (axial hintereinander angeordnete Spulenringe) 1 a, 1 b, 1 c, .... können zur Verringerung der ohmschen Verluste nur die Spulensegmente bestromt werden, die sich mit dem magnetischen Material 2 überdecken. Dabei kann bevorzugt vorgesehen sein, dass gleichzeitig die Spulensegmente im Bereich des magnetischen Materials 2 stromfrei geschaltet werden, in denen das magnetische Feld parallel zur Achse verläuft, also hier z.B. im Bereich des Permanentmagnetrings 2b. Diese individuelle Bestromung kann wiederum durch eine Steuerung mit evtl. Lageerfassung erfolgen, zu der dasselbe gilt, wie bei der Erfindung nach dem ersten Aspekt beschrieben wurde.

Durch einen lageabhängigen, kontinuierlichen Übergang zwischen stromlosem und bestromtem Zustand können die Feldkomponenten in radialer Richtung optimal zur Erzeugung einer Schubkraft ausgenutzt werden bei minimaler ohmscher Verlustleistung.

Durch die Wahl eines unterschiedlichen Längenverhältnisses von Magnetsegment (Ring 2a, 2b, ....) zu Spulensegment 1 a, 1b, ...wird auch der Streufluss optimal zur Krafterzeugung genutzt. Durch einzelne, unabhängige Bestromung der Spulensegmente werden alle zuvor aufgezählten Nachteile vermieden, so dass dieses Konzept bei Verwendung in einem Pumpantrieb das menschliche Herz in allen aufgeführten Aspekten nachempfindet.

Neben der Anpassung des Blutdrucks und des Volumenstroms während eines Pumpzyklus wird die aktive Ansaugung der Membran in der Diastole verhindert, indem der Membranstempel 3b des Kraftübertragungsglieds 3 nach Figur 1 mit der Membran 4a nicht fest verbunden ist. Demnach wird die Membran 4a nur durch den natürlichen Blutdruck zurückbewegt, sodass die Gefahr eines Kollabierens des Herzvorhofs nicht mehr besteht.

Die Geräuschentwicklung ist aufgrund der fehlenden maxwellschen Anziehungskräfte zwischen dem feststehenden und beweglichen Teil, der geringen Relativgeschwindigkeit zwischen den einzelnen Komponenten durch Einsatz eines Direktantriebs sowie der keinen Störkräften ausgesetzten Lagern minimal.

Die Lagerung von dem beweglichen Teil bzgl. dem feststehenden Teil kann auch hier beispielsweise über konventionelle Lager wie Kugel oder Gleitlager erfolgen oder vollständig verschleißfrei über hydrodynamische Lager.

Durch die segmentierte Bestrombarkeit der Spulen bei den Ausführungsformen nach beiden Aspekten ist auch eine erhöhte Redundanz des Antriebs gegeben. Ausfälle von Spulensegmenten oder Elektronikkomponenten führen nicht zum vollständigen Ausfall des Systems, sondern nur zu einer Verringerung der erreichbaren Fördermenge. Das Besondere an diesem Konzept bzgl. dem Stand der Technik ist, dass das erfindungsgemäße organunterstützende bzw. organersetzende System die Nachteile der aktuellen Systeme auf ein Minimum reduziert sowie die Vorteile der einzelnen Konzepte in einem einzigen System vereint.

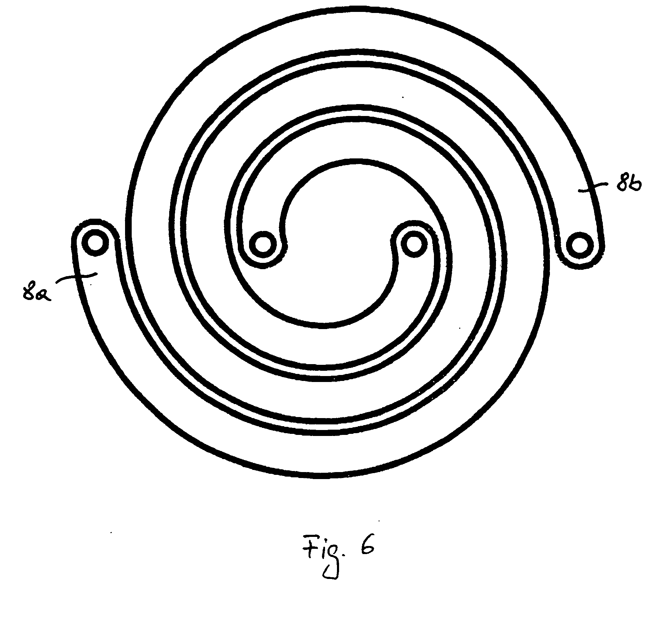

Für alle möglichen Ausführungen gilt, dass in dem Fall, wenn die Spulen den beweglichen Teil bilden, die Stromzuführung beispielsweise über Schleifkontakte, induktiv oder ein in Abbildung 6 gezeigtes Federsystem erfolgen kann. Hier sind als Beispiel lediglich zwei Leiter 8a und 8b dargestellt, die jeweils spiralförmig verlaufen und dabei einander umgeben, um einen elektrischen Kontakt von radial innen nach radial außen zu erzielen. Es kann hier also ein äußeres Ende der Leiter 8a und 8b an einem der Teile des Antriebs (z.B. Magnetteil) und das innere Ende am anderen Teil des Antriebs (z.B. Spulenteil) befestigt werden. In dem Fall, wenn die Spulen den ortsfesten Teil bilden, können diese Kontakte vollständig entfallen. So wird die Anzahl bewegter und verschleißbehafteter Komponenten weiter reduziert.

Durch die Integration eines Umrichters als Steuervorrichtung in den beweglichen Teil, z.B. die Spulenanordnung, kann die Anzahl der Federn auf 2 begrenzt werden, es sind jedoch pro Umrichter auch mehrere Federn möglich.

Durch die segmentierte Bestrombarkeit der Spulen ist eine erhöhte Redundanz des Antriebs gegeben. Ausfälle von Spulensegmenten oder Elektronikkomponenten führen nicht zum vollständigen Ausfall des Systems, sondern nur zu einer Verringerung des maximal erreichbaren Blutdrucks.

Bezüglich sämtlicher Ausführungen ist festzustellen, dass die in Verbindung mit einer Ausführung genannten technischen Merkmale nicht nur bei der spezifischen Ausführung eingesetzt werden können, sondern auch bei den jeweils anderen Ausführungen. Sämtliche offenbarte technische Merkmale dieser Erfindungsbeschreibung sind als erfindungswesentlich einzustufen und beliebig miteinander kombinierbar oder in Alleinstellung einsetzbar.

## Patentansprüche

1. Elektrischer Linearantrieb, insbesondere für ein Pumpsystem von einem Kunstherz, mit einem beweglichen und einem stationären Teil, bei dem der stationäre Teil durch eine Permanent-Magnetanordnung und der bewegliche Teil durch eine Spulenanordnung gebildet ist oder umgekehrt und bei dem die Spulenanordnung und die Permanent-Magnetanordnung relativ zueinander durch Bestromung der Spulenanordnung in einer axialen Richtung hin- und herbewegbar sind, die Spulenanordnung (1) in einem sich in einer axialen Richtung erstrekkenden Luftspalt (5) der Permanent-Magnetanordnung (2) angeordnet ist, deren magnetisches Material in axialer Richtung magnetisiert ist und die zumindest an ihren beiden axialen Enden innere und äußere Polschuhe (6a, 6b) aufweist, die einander in radialer Richtung und durch den Luftspalt (5) beabstandet gegenüberliegen, wodurch das magnetische Feld im Luftspalt (5) im Bereich der jeweils einander gegenüberliegenden Polschuhe (6a, 6b) in radialer Richtung konzentriert ist, wodurch wenigstens ein magnetischer Kreis ausgebildet ist, bei dem die Magnetfeldlinien sowohl durch den Luftspalt (5) radial von innen nach außen als auch durch den Luftspalt (5) von außen nach innen verlaufen, wobei die Permanentmagnetanordnung (2) in radialer Richtung auf beiden Seiten des Luftspaltes (5) zwischen den Polschuhen (6a, 6b) Permanentmagnete (2) aufweist, **dadurch gekennzeichnet, dass** die Permanent-Magnetanordnung (2) einen ersten inneren Permanentmagneten (2a) und einen koaxial dazu angeordneten und zur Bildung des Luftspaltes (5) beabstandeten zweiten äußeren, insbesondere im Querschnitt rahmen- oder ringförmigen Permanentmagneten (2b) aufweist.

2. Linearantrieb nach Anspruch 1, **dadurch gekennzeichnet, dass** zwischen den axial endseitigen Polschuhen (6a, 6b) wenigstens ein Paar von durch den Luftspalt (5) beabstandeten und in radialer Richtung einander gegenüberliegenden Polschuhe angeordnet ist, die das Magnetfeld in radialer Richtung konzentrieren.

3. Linearantrieb nach einem der vorherigen Ansprüche, **dadurch gekennzeichnet, dass** ein Permanentmagnet der Permanent-Magnetanordnung (2) in axialer Richtung wenigstens einen Polarisierungswechsel aufweist.

4. Vorrichtung nach einem der vorherigen Ansprüche, **dadurch gekennzeichnet, dass** die Dicke der Polschuhe (6a, 6b) in radialer Richtung auf den Luftspalt (5) zu zunimmt.

5. Vorrichtung nach einem der vorherigen Ansprüche, **dadurch gekennzeichnet, dass** die Spulenanordnung (1) wenigstens zwei in axialer Richtung hintereinander liegende und unterschiedlich bestrombare Spulensegmente (1a, 1b, 1c,...) aufweist, wobei, insbesondere durch eine übergeordnete Steuerung nur diejenigen Spulensegmente (1a, 1b, 1c,...) bestromt sind, die im Bereich der Polschuhe (6a, 6b) liegen.

6. Vorrichtung nach einem der vorherigen Ansprüche, **dadurch gekennzeichnet, dass** die Spulensegmente(1 a, 1 b, 1 c,...) und/oder Polschuhe (6a, 6b) in axialer Richtung unterschiedlich breit sind.

7. Vorrichtung nach Anspruch 6, **dadurch gekennzeichnet, dass** gleichzeitig die Spulensegmente (1a, 1b, 1c,...) stromfrei schaltbar sind, in denen das magnetische Feld nicht die genügende Stärke aufweist.

8. Vorrichtung nach Anspruch 7, **dadurch gekennzeichnet, dass** der Übergang zwischen stromlosem und bestromtem Zustand der Spulensegmente (1 a, 1 b, 1c,...) lageabhängig, insbesondere kontinuierlich einstellbar ist.

9. Vorrichtung nach Anspruch 8, **dadurch gekennzeichnet, dass** eine übergeordnete Steuerung vorgesehen ist, die eingerichtet ist, lageabhängig die Spulensegmente (1 a, 1 b, 1 c,...) zu bestromen.

10. Vorrichtung nach Anspruch 9, **dadurch gekennzeichnet, dass** zur Bestimmung der örtlichen Lage der Spulensegmente wenigstens eine Sensoranordnung vorgesehen ist, insbesondere eine Sensoranordnung mit einer Lichtschranke zur Feststellung einer Ursprungsposition und/oder eine Sensoranordnung zur insbesondere optischen Bestimmung einer Bewegungsrichtung und/oder Bewegungsweite.

11. Vorrichtung nach einem der vorherigen Ansprüche, **dadurch gekennzeichnet, dass** eine Stromversorgung der Spulenanordnung (1) ausgebildet ist durch wenigstens zwei elektrische Leiter (8a, 8b), die spiralförmig ausgebildet sind.

12. Vorrichtung nach Anspruch 11, **dadurch gekennzeichnet, dass** wenigstens zwei Leiter (8a, 8b) in einer Ebene einander spiralförmig umgeben.

13. Vorrichtung nach Anspruch 11 oder 12, **dadurch gekennzeichnet, dass** wenigstens zwei Leiter in einer Spirale geschichtet mit einer zwischenliegenden Isolierung angeordnet sind.

14. Vorrichtung nach Anspruch 13, **dadurch gekennzeichnet, dass** mehrere in einer Spirale geschichtete Leiter mit einem Versatz in Spiralrichtung beginnen und/oder enden.

15. Vorrichtung nach einem der vorherigen Ansprüche, **dadurch gekennzeichnet, dass** die Spulenanordnung (1) an einem Kraftübertragungselement (3) befestigt ist, das in einem mittleren Bereich, insbesondere koaxial in der Permanent-Meganetanordnung (2) gelagert ist und sich durch die Permanent-Magnetanordnung (2) hindurch erstreckt.

16. Vorrichtung nach Anspruch 15, **dadurch gekennzeichnet, dass** das Kraftübertragungselement (3) axial zu beiden Seiten der Permanent-Magnetanordnung (2) Druckplatten (3b) aufweist, mittels denen einen Kraft auf umgebende Elemente, insbesondere eine Membran (4a) einer Pumpkammer (4) ausübbar ist.

17. Pumpsystem, insbesondere Kunstherz, mit einem elektrischen Linearantrieb nach einem der vorherigen Ansprüche.

**Claims**

1. Electric linear drive, in particular for a pump system of an artificial heart, having a moving part and a stationary part, in which the stationary part is formed by a permanent-magnet arrangement and the moving part is formed by a coil arrangement, or vice versa, and in which the coil arrangement and the permanent-magnet arrangement can be moved to and fro in an axial direction relative to one another by current being applied to the coil arrangement, the coil arrangement (1) is arranged in an air gap (5), which extends in an axial direction, in the permanent-magnet arrangement (2), the magnetic material of the said permanent-magnet arrangement being magnetized in the axial direction and the said permanent-magnet arrangement having inner and outer pole shoes (6a, 6b) at least at its two axial ends, the said pole shoes being situated opposite one another in the radial direction and in a manner spaced apart by the air gap (5), as a result of which the magnetic field in the air gap (5) in the region of the pole shoes (6a, 6b) which are respectively situated opposite one another is concentrated in the radial direction, as a result of which at least one magnetic circuit is formed, the magnetic field lines in the said magnetic circuit running both radially from the inside to the outside through the air gap (5) and from the outside to the inside through the air gap (5), wherein the permanent-magnet arrangement (2) has permanent magnets (2) between the pole shoes (6a, 6b) on both sides of the air gap (5) in the radial direction, **characterized in that** the permanent-magnet arrangement (2) has a first, inner permanent magnet (2a) and a second, outer permanent magnet (2b), in particular of frame-like or ring-like cross section, which is arranged coaxially to the said first, inner permanent magnet and is spaced apart in order to form the air gap (5).

2. Linear drive according to Claim 1, **characterized in that** at least one pair of pole shoes, which are spaced apart by the air gap (5) and are situated opposite one another in the radial direction and concentrate the magnetic field in the radial direction, is arranged between the axially end-side pole shoes (6a, 6b).

3. Linear drive according to either of the preceding claims, **characterized in that** a permanent magnet of the permanent-magnet arrangement (2) exhibits at least one change in polarization in the axial direction.

4. Apparatus according to one of the preceding claims, **characterized in that** the thickness of the pole shoes (6a, 6b) increases in the radial direction towards the air gap (5).

5. Apparatus according to one of the preceding claims, **characterized in that** the coil arrangement (1) has at least two coil segments (1a, 1b, 1c, ...) which are situated one behind the other in the axial direction and to which different currents can be applied, wherein current is applied, in particular by a superordinate control system, only to those coil segments (1a, 1b, 1c, ...) which are situated in the region of the pole shoes (6a, 6b).

6. Apparatus according to one of the preceding claims, **characterized in that** the coil segments (1a, 1b, 1c, ...) and/or pole shoes (6a, 6b) have different widths in the axial direction.

7. Apparatus according to Claim 6, **characterized in that** the coil segments (1a, 1b, 1c, ...) in which the magnetic field is not strong enough can be switched to be free of current at the same time.

8. Apparatus according to Claim 7, **characterized in that** the transition between the state in which the coil segments (1a, 1b, 1c, ...) are currentless and the state in which current is applied to the said coil segments can be adjusted in a position-dependent manner, in particular continuously.

9. Apparatus according to Claim 8, **characterized in that** a superordinate control system is provided, the said superordinate control system being designed to apply current to the coil segments (1a, 1b, 1c, ...) in a position-dependent manner.

10. Apparatus according to Claim 9, **characterized in that** at least one sensor arrangement is provided for the purpose of determining the local position of the coil segments, in particular a sensor arrangement having a light barrier for establishing a starting position and/or a sensor arrangement for, in particular, optically determining a direction of movement and/or extent of movement.

11. Apparatus according to one of the preceding claims, **characterized in that** current is supplied to the coil arrangement (1) by at least two electrical conductors (8a, 8b) which are designed in the form of a spiral.

12. Apparatus according to Claim 11, **characterized in that** at least two conductors (8a, 8b) surround one another in the form of a spiral in one plane.

13. Apparatus according to Claim 11 or 12, **characterized in that** at least two conductors are arranged in a spiral in a manner layered with an interposed insulation means.

14. Apparatus according to Claim 13, **characterized in that** a plurality of conductors which are layered in a spiral begin and/or end with an offset in the spiral direction.

15. Apparatus according to one of the preceding claims, **characterized in that** the coil arrangement (1) is attached to a force-transmitting element (3) which is mounted in a central region, in particular coaxially, in the permanent-magnet arrangement (2) and extends through the permanent-magnet arrangement (2).

16. Apparatus according to Claim 15, **characterized in that** the force-transmitting element (3) has pressure plates (3b) axially on both sides of the permanent-magnet arrangement (2), it being possible for a force to be exerted on surrounding elements, in particular a diaphragm (4a) of a pumping chamber (4), by means of the said pressure plates.

17. Pump system, in particular artificial heart, having an electric linear drive according to one of the preceding claims.

**Revendications**

1. Mécanisme d'entraînement linéaire électrique, notamment pour un système de pompage d'une résine synthétique, comprenant une partie mobile et une partie fixe, avec lequel la partie fixe est formée par un arrangement d'aimants permanents et la partie mobile par un arrangement de bobines ou inversement et avec lequel l'arrangement de bobines et l'arrangement d'aimants permanents peuvent effectuer un mouvement de va-et-vient dans une direction axiale l'un par rapport à l'autre en alimentant l'arrangement de bobines, l'arrangement de bobines (1) étant disposé dans un entrefer (5) de l'arrangement d'aimants permanents (2) qui s'étend dans une direction axiale, dont le matériau magnétique est magnétisé dans la direction axiale et qui présente au moins à ses deux extrémités axiales des pièces polaires (6a, 6b) intérieure et extérieure, lesquelles sont opposées dans la direction radiale et espacées par l'entrefer (5), ce qui a pour effet que le champ magnétique dans l'entrefer (5) est concentré dans la direction radiale dans la zone des pièces polaires (6a, 6b) respectivement opposées l'une à l'autre, ce qui forme au moins un circuit magnétique avec lequel les lignes de champ magnétique s'étendent à la fois à travers l'entrefer (5) dans la direction radiale de l'intérieur vers l'extérieur et à travers l'entrefer (5) de l'extérieur vers l'intérieur, l'arrangement d'aimants permanents (2) présentant des aimants permanents (2) des deux côtés de l'entrefer (5) entre les pièces polaires (6a, 6b), **caractérisé en ce que** l'arrangement d'aimants permanents (2) présente un premier aimant permanent intérieur (2a) et un deuxième aimant permanent (2b) extérieur, notamment de section transversale en forme de cadre ou d'anneau, disposé de manière coaxiale par rapport à celui-ci et espacé afin de former l'entrefer (5).

2. Mécanisme d'entraînement linéaire selon la revendication 1, **caractérisé en ce qu'**entre les pièces polaires (6a, 6b) du côté des extrémités axiales est disposée au moins une paire de pièces polaires espacées par l'entrefer (5) et opposées l'une à l'autre dans la direction radiale, lesquelles concentrent le champ magnétique dans la direction radiale.

3. Mécanisme d'entraînement linéaire selon l'une des revendications précédentes, **caractérisé en ce qu'**un aimant permanent de l'arrangement d'aimants permanents (2) présente dans la direction axiale au moins un changement de polarisation.

4. Dispositif selon l'une des revendications précédentes, **caractérisé en ce que** l'épaisseur des pièces polaires (6a, 6b) dans la direction radiale augmente en direction de l'entrefer (5).

5. Dispositif selon l'une des revendications précédentes, **caractérisé en ce que** l'arrangement de bobines (1) présente au moins deux segments de bobine (1a, 1b, 1c, ...) disposés l'un derrière l'autre dans la direction axiale et pouvant être alimentés électriquement différemment, seuls étant alimentés électriquement les segments de bobine (1a, 1b, 1c, ...), notamment par une commande de supervision, qui se trouvent dans la zone des pièces polaires (6a, 6b).

6. Dispositif selon l'une des revendications précédentes, **caractérisé en ce que** les segments de bobine (1a, 1b, 1c, ...) et/ou les pièces polaires (6a, 6b) présentent des largeurs différentes dans la direction axiale.

7. Dispositif selon la revendication 6, **caractérisé en ce que** les segments de bobine (1a, 1b, 1c, ...) dans lesquels le champ magnétique ne présente pas l'intensité suffisante peuvent être mis hors tension simultanément.

8. Dispositif selon la revendication 7, **caractérisé en ce que** la transition entre l'état non alimenté et l'état alimenté électriquement des segments de bobine (1a, 1b, 1c, ...) est réglable en fonction de la position, notamment continuellement.

9. Dispositif selon la revendication 8, **caractérisé en ce qu'**il est prévu une commande de supervision qui est conçue pour alimenter électriquement les segments de bobine (1a, 1b, 1c, ...) en fonction de la position.

10. Dispositif selon la revendication 9, **caractérisé en ce qu'**au moins un arrangement de détecteurs est prévu pour déterminer la position des segments de bobine, notamment un arrangement de détecteurs comprenant une barrière photoélectrique pour définir une position d'origine et/ou un arrangement de détecteurs destiné à la détermination notamment optique d'un sens de déplacement et/ou d'une amplitude de déplacement.

**11.** Dispositif selon l'une des revendications précédentes, **caractérisé en ce qu'**une alimentation électrique de l'arrangement de bobines (1) est formée par au moins deux conducteurs électriques (8a, 8b) qui sont réalisés en forme de spirale.

**12.** Dispositif selon la revendication 11, **caractérisé en ce qu'**au moins deux conducteurs (8a, 8b) s'entourent mutuellement en forme de spirale dans un plan.

**13.** Dispositif selon la revendication 11 ou 12, **caractérisé en ce qu'**au moins deux conducteurs sont disposés en une spirale empilés avec une isolation interposée entre eux.

**14.** Dispositif selon la revendication 13, **caractérisé en ce que** plusieurs conducteurs empilés en une spirale commencent et/ou se terminent avec un décale dans le sens de la spirale.

**15.** Dispositif selon l'une des revendications précédentes, **caractérisé en ce que** l'arrangement de bobines (1) est fixé à un élément de transmission de force (3) qui est logé dans une zone centrale, notamment de manière coaxiale, dans l'arrangement d'aimants permanents (2) et s'étend à travers l'arrangement d'aimants permanents (2).

**16.** Dispositif selon la revendication 15, **caractérisé en ce que** l'élément de transmission de force (3) présente dans le sens axial, vers les deux côtés de l'arrangement d'aimants permanents (2), des plaques de compression (3b) au moyen desquelles une force peut être exercée sur les éléments environnants, notamment une membrane (4a) d'une chambre de pompe (4).

**17.** Système de pompage, notamment d'une résine synthétique, comprenant un mécanisme d'entraînement linéaire électrique selon l'une des revendications précédentes.

Fig. 1

Fig. 2a

Fig. 2b

Fig. 2c

EP 2 178 581 B1

Fig. 3

Fig. 4

Fig. 5

8b

8a

Fig. 6

## IN DER BESCHREIBUNG AUFGEFÜHRTE DOKUMENTE

**In der Beschreibung aufgeführte Patentdokumente**

- DE 10360713 A1
- US 5300111 A
- US 5360445 A
- US 5924975 A
- US 6194796 B1
- US 6190409 B1